**Europäisches Patentamt**

**European Patent Office**

**Office européen des brevets**

(19)

(11) Veröffentlichungsnummer: **0 211 386**
**A2**

(12) # EUROPÄISCHE PATENTANMELDUNG

(21) Anmeldenummer: 86110495.8

(22) Anmeldetag: 30.07.86

(51) Int. Cl.⁴: **G 01 N 27/02**
**G 01 N 33/04**

(30) Priorität: 30.07.85 HU 292285

(43) Veröffentlichungstag der Anmeldung:
25.02.87 Patentblatt 87/9

(84) Benannte Vertragsstaaten:
AT BE CH DE FR GB IT LI LU NL SE

(71) Anmelder: Mezögazdasági Föiskola Kaposvár
Denes major 2
H-7401 Kaposvár(HU)

(72) Erfinder: Takatsy, Tibor, Dr.
Buzuvirag u. 17
Kaposvar(HU)

(72) Erfinder: Gyánó, József
Füredi ut 10
Kaposvár(HU)

(72) Erfinder: Kapitány, Ferenc
Hunyadi u. 28
Kaposmérö(HU)

(72) Erfinder: Munkácsi, Gyula
Balogh A. u. 12
Kaposvár(HU)

(72) Erfinder: Fehér, István, Dr.
Szántó I. u. 18
Kaposvár(HU)

(74) Vertreter: Lehn, Werner, Dipl.-Ing. et al,
Hoffmann, Eitle & Partner Patentanwälte Arabellastrasse
4 (Sternhaus)
D-8000 München 81(DE)

(54) Verfahren und Vorrichtung zum Nachweis von Euterentzündung.

(57) Die Erfindung betrifft ein Verfahren und eine Vorrichtung zum Nachweis von Euterentzündung.

Das Wesen des erfindungs gemässen Verfahrens besteht darin. dass den vier Eutervierteln Milchproben entnommen werden, der über die Milchprobe fliessende Strom und die über der Milchprobe abfallende Spannung gemessen werden, anhand der gemessenen Werte durch Quotientenbildung die entsprechenden Impedanzwerte ermittelt werden, welche gespeichert werden, die gleichen Operationen für alle vier Milchproben durchgeführt werden und daraus der maximale Impedanzwert gesucht wird, danach die Quotienten der Impedanzen der übrigen Milchproben zur maximalen Impedanz gebildet werden und diese Quotienten mit einem solchen Grenzwert verglichen werden, dessen Wert in Abhängigkeit vom Impedanzwert der Milchprobe mit maximaler Impedanz im voraus auf die Weise bestimmt wurde, dass die möglichen Impedanzwerte in mehrere Bereiche aufgeteilt werden und jedem Bereich ein experimentell ermittelter Grenzimpedanzquotient zugeordnet wird, wobei die Milchprobe, deren Impedanzquotient geringer als der den einzelnen Bereichen zugeordnete Grenzimpedanzquotient ist, als mit Euterentzündung behaftete bewertet wird.

Die erfindungsgemässe Vorrichtung enthält mindestens eine Meßsonde (2), eine elektronische Einheit und eine An-zeigeeinheit (7), wobei die elektronische Einheit mit einem Generator (1), einem Messverstärker (4), einem zwischen den Meßsonden und dem Messverstärker (4) eingefügten Messpunktschalter (3), einem A/D-Wandler (5), und einer Steuereinheit (6) versehen ist, wobei die Meßsonden (2) mit dem Ausgang des Generators (1), ein Ausgang des Messverstärkers (4) mit dem Eingang des A/D-Wandlers (5), digitale Ausgänge des A/D-Wandlers (5) mit Eingängen der Steuereinheit (6), Steuerausgänge der Steuereinheit (6) mit Steuereingängen der Messpunktschalter (3) und Eingängen der Anzeigeeinheit (7) verbunden sind.

Fig.1

VERFAHREN UND VORRICHTUNG ZUM NACHWEIS VON EUTERENTZÜNDUNG

Die Erfindung betrifft ein Verfahren zum Nachweis von Mastitis (Euterentzündung), bei welchem die elektrische Leitfähigkeit der aus allen vier Euterviertein des geprüften Subjektes entnommenen Milch gemessen wird, danach die auf diese Weise erhaltenen Werte mit einem vorgegebenen Wert verglichen werden oder durch Teilung der erhaltenen Impedanzwerte durch einen maximalen Impedanzwert ein Quotient gebildet wird, die erhaltenen Quotienten mit einem vorgegebenen Wert verglichen werden.

Die Erfindung betrifft weiterhin eine elektronische Vorrichtung zur Durchführung des Verfahrens, welche mindestens eine Meßsonde, eine elektronische Einheit und eine Anzeigeeinheit aufweist.

Es ist bekannt, dass sich bei der grossbetrieblichen Tierhaltung eine äusserst hohe Tierkonzentration ausbildet. Dieser Umstand und der der maschinellen Melkung führte zur bedeutenden Erhöhung der Anzahl derjenigen Tiere, welche an Mastitis (Euterendzündung) leiden.

Es gibt Länder, in denen die Anzahl der an Mastitis leidenden Tiere 40% des gesamten Rinderbestandes beträgt. Voraussetzung für einen erfolgreichen Schutz dagegen ist eine frühe und zuverlässige Erkennung der Euterentzündung. Es ist wünschenswert, dass die Krankheit schon in ihrem subklinischen Stadium erkannt wird. Es ist weiterhin wichtig, dass die Prüfungsmethode und das Prüfungsmittel einfach aufgebaut, leicht bedienbar und zuverlässig sind und darüberhinaus eine schnelle Sicherung des Resultates gewährleisten.

Es wurde erkannt, dass aus der elektrischen Leitfähigkeit der Milch auf die Mastitis geschlussfolgert werden kann. Später wurde dann erkannt, dass ein besseres Resultat dann erreicht werden kann, wenn die elektrische Leitfähigkeiten der aus den Euterviertein

je eines Tieres entnommenen Milchprobe verglichen werden, als wenn konstante Wertgrenzen in dem möglichen Bereich der elektrischen Leitfähigkeit zur Bestimmung der Kategorien hinsichtlich der Mastitis gewählt werden.

Es sind sogar mehrere Varianten von Vergleichsmethoden bekannt geworden. Bei der einen Vergleichsmethode wird die elektrische Leitfähigkeit der den vier Euterviertel eines Tieres entnommenen Milchproben bestimmt, danach wird die Milchprobe, welche den kleinsten Wert der elektrischen Leitfähigkeit aufweist, ausgewählt, danach werden die Werte der elektrischen Leitfähigkeit für die den übrigen Eutervierteln entnommenen Milchproben und die Differenzen zu dem erwähnten kleinsten Wert gebildet. Sollte irgendein Differenzwert einen bestimmten konstanten Differenzwert überschreiten, wird die an dem Vergleich teilnehmende Milchprobe mit grösserer elektrischer Leitfähigkeit als mit Mastitis behaftete bewertet.

Bei einer anderen Vergleichsmethode wird auf obenerwähnte Weise von den aus den einzelnen Eutervierteln entnommenen Milchproben die die kleinste elektrische Leitfähigkeit aufweisende Milchprobe ermittelt. In Hinsicht auf die Werte der elektrischen Leitfähigkeit dieser Milchprobe und der aus den anderen Eutervierteln stammenden Milchproben werden jedoch anstelle der Differenzen die Quotienten der Werte der elektrischen Leitfähigkeit gebildet. Sollte irgendein Quotient einen im voraus festgelegten Wert überschreiten, so wird von den am Vergleich teilnehmenden Milchprobem die mit grösserer elektrischer Leitfähigkeit als mit Mastitis behaftete bewertet.

Eine dritte Methode ergänzt die obenerwähnten zwei Methoden damit, dass die Milchprobe - alle Milchproben - deren Wert der elektrischen Leitfähigkeit über einen bestimmten Wert liegt, als mit Mastitis behaftete bewertet wird.

Die zum Nachweis von Mastitis dienenden, auf der Messung der elektrischen Leitfähigkeit der Milch beruhenden bekannten Vorrichtungen liefern auf der Grundlage von zwei vorgegebenen Leitfähigkeitswerten durch den mit diesen Werten erfolgenden Vergleich Richtlinien für die Bewertung. Im allgemeinen dienen die mittels zwei Leuchtdioden erzeugten Anzeigen als Grundlage für die Bewertung. Die bekannten Vorrichtungen weisen einen Prüfkelch zur Aufnahme der geprüften Milchproben auf und als Ergebnis der Prüfung setzt sich entweder eine grüne Leuchtdiode oder eine rote Leuchtdiode oder beide in Betrieb.

Die grüne Anzeige meldet die in Hinsicht auf die Mastitis negativen Ergebnisse der Milchprobe, während die rote Anzeige einen Hinweis auf das positive Resultat der Milchprobe gibt, wenn jedoch beide, verschiedenenfarbigen Leuchtdioden gleichzeitig funktionieren, weist diese Anzeige darauf hin, dass die geprüfte Milchprobe positiv verdächtig ist. Da bei der Verwendung der bekannten, zum Nachweis von Mastitis dienenden Vorrichtungen die Bewertung der Milchprobe anhand von zwei konstanten Wertgrenzen der Leitfähigkeit erfolgt, sind derartige Vorrichtungen zur Vergleichsprüfung von aus vier Eutervirteln stammenden vier Milchproben nur in einem äusserst begrenzten Mass geeignet.

Die Nachteile der bekannten Vorrichtungen und der mittels dieser durchgeführten Verfahren haben verschiedene Ursachen. So ändert sich z.B. die elektrische Leitfähigkeit der Milch nicht nur infolge der Euterentzündung, sondern hängt von einer Reihe anderer Faktoren ab. Derartige Faktoren sind z.B. die Zusammensetzung des Futters, die vorgeschrittene Trächtigkeit, ein langer Melkzyklus, das Alter der Kuh, usw. Unter diesen Umständen ist der Nachweis von Mastitis anhand konstanter Werte der elektrischen Leitfähigkeit der Milch nicht mit befrädigender Sicherheit durchführbar. Den in Frage kommenden Wertbereich
der elektrischen Leitfähigkeit der Milch mit zwei konkreten Werten in drei Teile zu teilen, stellt eine äusserst
grobe Kategorisierung dar, bei welcher kein zuverlässiges Resultat zu erwarten ist.

Bei Anwendung der erfindungsgemässen Vorrichtung sind die angeführten Mängel, Nachteile grösstenteils beseitigbar, umgehbar. Die Neuartigkeit der erfindungsgemässen Vorrichtung beruht auf zwei Erkenntnissen. Eine der Erkenntnisse ist, dass das Material
und die Form - die Geometrie - der Fühler der bei der
Messung verwendeten Sonde die Messungsergebnisse bedeutend beeinflusst. Das Wesen der anderen Erkenntnis
liegt darin, dass anhand des Vergleichens der Impedanzen der vier Euterviertein entnommenen Milchproben
miteinander die zuverlässigste Entscheidung so getroffen werden kann, wenn der Grenzquotient in Abhängigkeit vom Impedanzwert der Milchprobe mit maximaler Impedanz festgelegt wird.

Das gestellte Ziel kann durch die Erfindung
im allgemeinsten Sinne durch ein Verfahren erreicht
werden, bei welchem die Impedanzmessung in zwei Schritten durchgeführt wird, und zwar auf die Weise, dass
der über die Sonde fliessende Strom und die an der Sonde abfallende Spannung gemessen werden, danach mittels
einer zur Durchführung von arithmetischen Operationen
ebenfalls geeigneten Steuerelektronik der Quotient der
gemessenen Werte gebildet wird und die auf diese Weise bestimmten Impedanzwerte gespeichert werden, danach
werden die gleichen Operationen für alle den vier Euterviertein entnommenen Milchproben durchgeführt und
davon der maximale Impedanzwert gesucht, danach wird
der Quotient der Impedanzen der übrigen Milchproben
zur maximalen Impedanz gebildet. Die auf diese Weise erhaltenen Quotienten werden mit einem solchen Grenzwert verglichen, dessen Wert in Abhängigkeit vom Impedanzwert der Milchprobe mit maximaler Impedanz im voraus festgelegt wurde, und zwar auf die Weise, dass die
möglichen Impedanzwerte in mehrere Bereiche aufgeteilt
werden und jedem Bereich ein experimentell  bestimmter
Impedanzgrenzwert zugeordnet wird.

Es ist vorteilhaft, das erfindungsgemässe Verfahren vor dem Aufsetzen der Melkmaschine
durchzuführen, da nur auf diese Weise gewährleistet
werden kann, dass die aus dem entzündeten Euter abgemolkene Milch sich nicht mit der aus den gesunden Eutern
abgemolkenen Milch vermischt und dadurch die Qualität
der Milch des gesamten Milchbehälters und die nachfolgende Aufbereitbarkeit der Milch verschlechtert.

Das erfindungsgemässe Verfahren kann auf
allgemeinste Weise mittels einer solchen Vorrichtung durchgeführt werden, bei welcher die elektronische Einheit
einen ein Wechselspannungssignal erzeugenden Generator,
einen Messverstärker, einen zwischen den Meßsonden und
dem Messverstärker eingefügten Messpunktschalter - vorzugsweise einen Analogschalter - einem das analoge Signal des  Messverstärkers in ein digitales Signal verwandelnder A/D-Wandler (Analog-Digital-Wandler) und eine
Steuereinheit - vorzugweise Mikroprozessorsteuereinheit -
aufweist, wobei die Meßsonden mit dem Ausgang des Generators verbunden sind, der ein analoges Signal liefernde
Ausgang des Messverstärkers an den Eingang des A/D-Wandlers angeschlossen ist, desweiteren die digitalen Ausgänge des A/D-Wandlers mit den Eingängen der Mikroprozessorsteuereinheit verbunden sind, während die Steuerausgänge der Mikroprozessorsteuereinheit an Steuereingänge
der Messpunktschalter und an Eingänge einer Anzeigeeinheit
geführt sind.

Bei einer bevorzugten Ausführungsform der
Erfindung ist die Meßsonde mit einem aus Isolationsmaterial, vorzugsweise aus Kunststoff bestehenden Messkelch und in dem Messkelch befestigten Elektroden ausgebildet, wobei der Messkelch von einer Kelchseite und einem Kelchboden begrenzt ist, desweiteren zwischen der Kelchseite und dem Kelchboden eine Ringelektrode befestigt ist, während in dem Kelchboden eine, im wesentlichen zur Ebene der Ringelektrode quergerichtete, in deren Achsenlinie befindliche Stiftelektrode befestigt ist.

Bei dieser Ausführungsform der Erfindung ist es von Vorteil, wenn der aus Kunststoff bestehende Messkelch aus zwei, sich einander gewindeartig anpassenden Teilen ausgebildet ist.

Durch diese Ausführungsform wird gewährleistet, dass die Meßsonde schnell zusammengestellt und auseinandermontiert werden kann.

Nachstehend wird die Erfindung anhand von bevorzugten Ausführungsbeispielen unter Bezugnahme auf die beigefügte Zeichnung näher erläutert. In der Zeichnung zeigen:

Fig. 1          ein vereinfachtes Blockschema der erfindungsgemässen Vorrichtung zum Nachweis von Euterentzündung,

Fig. 2          ein Prizipschaltschema der Ausführungsbeispiele der in der Ausführungsform gemäss Fig. 1 verwendeten Generator, Meßsonden und Messpunktschalter,

Fig. 3          ein Prinzipschaltschema einer Ausführungsform des in der Vorrichtung gemäss Fig. 1 verwendeten Messverstärkers,

Fig. 4          ein Prinzipschaltschema einer Ausführungsform des in der Vorrichtung gemäss Fig. 1 verwendeten Analog/Digital-Wandlers,

Fig. 5          ein Prinzipschaltschema eines Ausführungsbeispieles der in der Vorrichtung gemäss Fig. 1 verwendeten Mikroprozessorsteuereinheit, und

Fig. 6          einen Schnitt durch ein bevorzugtes Ausführungsbeispiel der in der erfindungsgemässen Vorrichtung verwendeten Meßsonde.

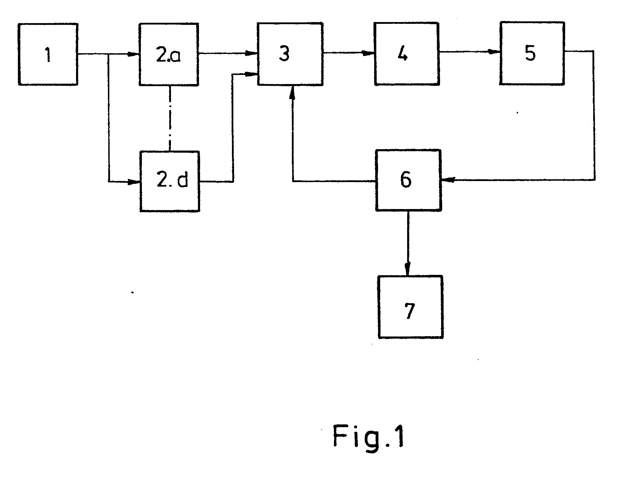

In Fig. 1 sind die Haupteinheiten der erfindungsgemässen Vorrichtung zum Nachweis von Euterentzündung und die Verbindungen zwischen diesen veranschaulicht. Ein Ausgang eines zur Erzeugung eines Wechselspannungssignals dienenden Generators 1 ist mit Meßsonden 2a, 2b, 2c und 2d verbunden, welche zur gleichzeitigen Aufnahme von den Eutervierteln abgemolkenen vier Milchproben dienen. Die Ausgänge der Meßsonden 2 sind an Eingänge eines Messpunktschalters 3 angeschlossen, während der Ausgang des Messpunktschalters 3 mit dem Eingang eines Messverstärkers 4 verbunden ist. Ein ein analoges Signal erzeugender Ausgang des Messverstärkers 4 ist an einen Analogeingang eines A/D-Wandlers 5 geführt, während ein Digitalausgang des A/D-Wandlers 5 an eine mit einem Mikroprozessor ausgebildete Steuereinheit 6 angeschlossen ist. Die Steuerausgänge der Steuereinheit 6 sind mit dem Messpunktschalter 3 und einer Anzeigeeinheit 7 verbunden.

Die Funktion der erfindungsgemässen, zum Nachweis von Euterentzündung dienenden Vorrichtung wird durch ein in dem Speicher der mit einem Mikroprozessor ausgebildeten Steuereinheit 6 gespeichertes Programm gesteuert. Der Generator 1 erzeugt ein Wechselspannungssignal, auf dessen Wirkung über die in die Meßsonden 2 eingemolkenen Milchproben ebenfalls ein Wechselstrom fliesst, bzw. über diese eine Spannung abfällt. Der Messpunktschalter 3 schaltet entsprechend dem Programm der Steuereinheit 6 nacheinander ein zu dem vom Ausgang der einzelnen Meßsonden abgenommenen Strom, bzw. Spannung proportionales Meßsignal an den Eingang des Messverstärkers 4. Das von dem Messverstärker 3 erzeugte analoge Gleichstromsignal wird von dem A/D-Wandler 5 in ein mittels der Steuereinheit 6 verarbeitbares digitales Signal verwandelt.

Die mit einem Mikroprozessor ausgebildete Steuereinheit 6 speichert die einzelnen Messergebnisse und errechnet aus den von den einzelnen Meßsonden ankommenden Strom- und Spannungswerten die Impedanz der Milchprobe. Die Steuereinheit 6 ermittelt durch synchrone Steuerung des Messpunktschalters die Impedanz für alle vier Milchproben, sucht die Milchprobe mit maximaler Impedanz und bildet den Quotienten der Impedanzen der übrigen Milchproben zu der Impedanz der Milchprobe, deren Impedanz als maximale bestimmt wurde.

Darüberhinaus bestimmt die Steuereinheit 6, in welchen der experimentell im voraus bestimmten Impedanzbereiche der Impedanzwert der Milchprobe mit maximaler Impedanz fällt. Mittels dieser Steuereinheit wird der Grenzwert des dem auf diese Weise ausgewählten Impedanzbereich zugeordneten, ebenfalls experimentell im voraus bestimmten Impedanzquotienten mit den berechneten, auf die maximale Impedanz bezogenen Impedanzquotienten verglichen und als Ergebnis des Vergleiches entschieden, ob die aus dem geprüften Euterviertel stammende Milchprobe mit Euterentzündung behaftet ist oder nicht.

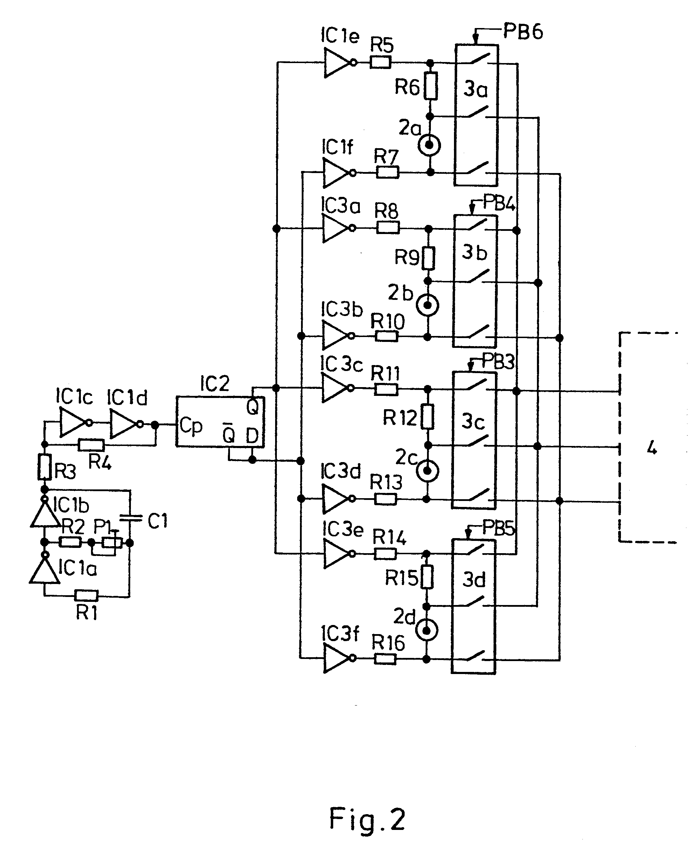

Wie aus Fig. 2 ersichtlich ist, ist der Generator 1 mit Invertern IC1a-IC1d, Widerständen R1-R4 und einem D-Speicher IC2 ausgebildet. Der Ausgang des Rechtecksignale erzeugenden Generators 1 ist über Inverter IC1e-IC1f und IC3a-IC3f und Widerstände R5-R16 mit den Meßsonden 2a-2d verbunden. Von jeder einzelnen als Widerstandsteilerglied eingebauten Meßsonde sind drei Leitungen an den Eingang der Messpunktschalter 3a-3d geführt, während der Ausgang der Messpunktschalter derart vereinigt ist, dass die drei Anschlüsse der Messpunktschalter an je einen Punkt eingebunden sind, d.h. am Ausgang des Messpunktschalters sind drei Leitungen von jeweils nur
einer Meßsonde zu sehen. Die Messpunktschalter sind
vorzugsweise mit Analogschaltern ausgebildet und ihre
entsprechende Funktion wird durch das Programm der
Steuereinheit 6 gesichert.

Der aus den vier Invertern des IC1 ausgebildete Oszillator ist ein Oszillator mit einer
Frequenz von 3,2 kHz, sein rechteckiges Ausgangssignal wird von dem D-Speicher IC2 geteilt und symmetrisiert. Der zur Messung der Impedanz der Milchproben
erforderliche Strom wird von den mittels der Inverter
IC1e-IC1f und IC3a-IC3f ausgebildeten Antriebsorganen
bereitgestellt. Die zu dem Strom, welcher über die in
den Meßsonden befindlichen Milchproben fliesst, proportionale Spannung kann von den beiden Anschlüssen
der Widerstände R6, R9, R12, R15 abgenommen werden,
während die über den Meßsonden fallende Spannung von
den an den beiden Elektroden der Meßsonde angeschlossenen Klemmen abgenommen werden kann.

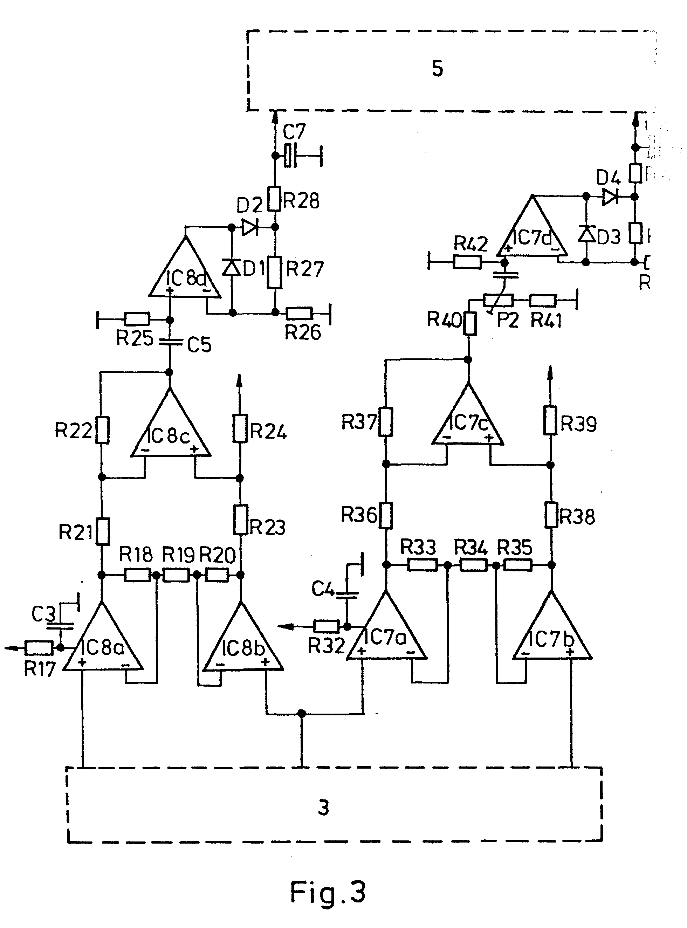

In Fig. 3 ist der Messverstärker der erfindungsgemässen Vorrichtung näher veranschaulicht,
welcher mit zwei annähernd gleich aufgebauten Verstärkerketten ausgebildet ist. Die eine Verstärkerkette wird
durch die Operationsverstärker IC7a-IC7d und die andere durch die Operationsverstärker IC8a-IC8d gebildet.
Der Ausgang des Operationsverstärkers IC7c ist abweichend
vom Ausgang des Operationsverstärkers IC8c, welcher über
einen Kopplungskondensator C5 an den nicht-invertierenden Eingang des Operationsverstärkers IC8d angeschlossen ist, über einen mit den Gliedern R40, P2 und R41
ausgebildeten Spannungsteiler und einen Kopplungskondensator an den nicht-invertierenden Eingang des Operationsverstärkers IC7d geführt. Die Operationsverstärker IC7d und IC8d bilden mit den in ihren Rückkopplungskreis eingefügten Dioden einen Spitzengleichrichter.

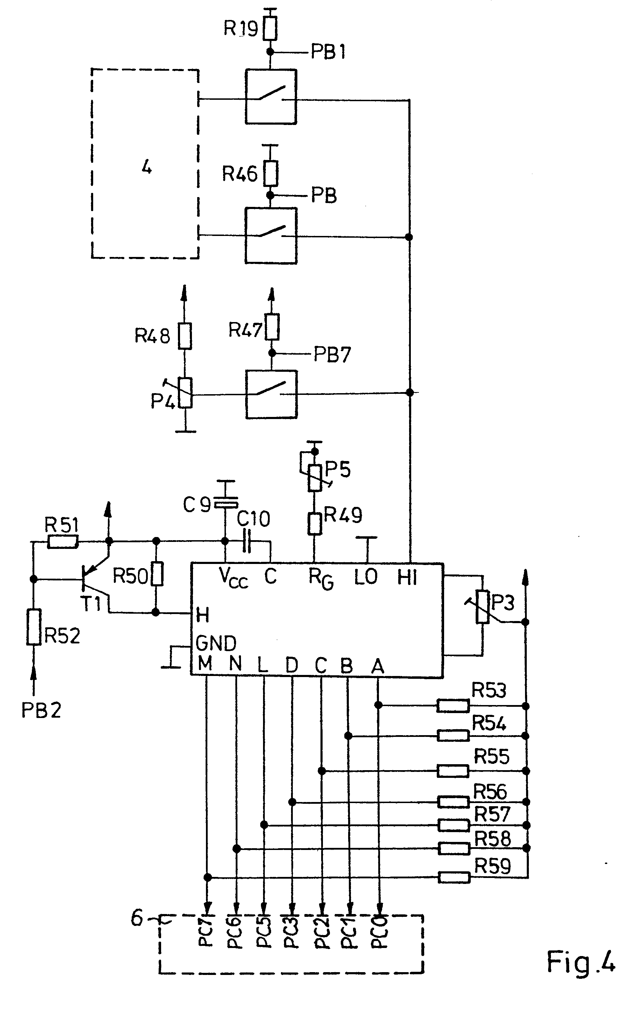

Der mit den Operationsverstärkern IC8 ausgebildete Verstärker verstärkt die zu dem über die Meßsonde, welche durch den Messpunktschalter ausgewählt wird, fliessenden Strom proportionale Spannung, während der mit den Operationsverstärkern IC7 ausgebildete Verstärker die über die ausgewählte Meßsonde fallende Spannung verstärkt, wodurch an den beiden Ausgängen des Messverstärkers 4 ein konstantes, für die Impedanz der in die Meßsonde gemolkenen Milchprobe charakteristisches analoges Signal erscheint, welches durch den in Fig. 4 näher dargestellten A/D-Wandler in ein digitales Signal verwandelt wird.

An den Analogeingang HI des in Fig. 4 dargestellten A/D-Wandlers sind die beiden Ausgangssignale des Meßverstärkers 4 und das mittels der Glieder R48 und P4 geteilte Signal der Speisespannung über je einen Analogschalter geführt. Bei der vorliegenden Ausführungsform wurde beispielsweise ein A/D-Wandler des Types C 520 D verwendet in einer vom Hersteller vorgeschlagenen Schaltung, das schliesst jedoch nicht die Möglichkeit der Anwendung anderer A/D-Wandler aus. Die Steuerung der Analogschalter und des das Haltesignal (hold) schaltenden Transistors T1 wird durch die Steuerausgänge der Steuereinheit 6 anhand eines in der Steuereinheit 6 gespeicherten Programmes durchgeführt. Während der Messung wird jede einzelne Meßsonde einmal über den Messpunktschalter mit den Eingängen des Messverstärkers verbunden. Der Messverstärker misst dabei eine zum über die Meßsonde fliessenden Strom proportionale Spannung und die über der Meßsonde abfallende Spannung und dementsprechend erscheinen die beiden Messergebnisse gleichzeitig an seinen beiden Ausgängen. Diese beiden Ausgänge schliessen sich bei jeder Sondenauswahl über je einen Analogschalter nacheinander an den Analogeingang HI des A/D-Wandlers an. An den Analogeingang HI des A/D-Wandlers wird weiterhin - ebenfalls über einen Analogschalter - ein den Wert der Speisespannung überprüfendes Signal geleitet. Die Analogschalter werden von den Steuerausgängen der mit einem Mikroprozessor ausgebildeten Steuereinheit 6 anhand eines Messprogrammes gesteuert. Sollte die Speisespannung zur Durchführung einer genauen Messung nicht ausreichend sein, so lässt das Programm an der Anzeigeeinheit 7 eine die Speisespannung überprüfende LED-Diode aufleuchten und sperrt die Messungen solange, bis zum Beispiel durch Aufladen der Akkumulatoren die entsprechende Speisespannung erreicht wird.

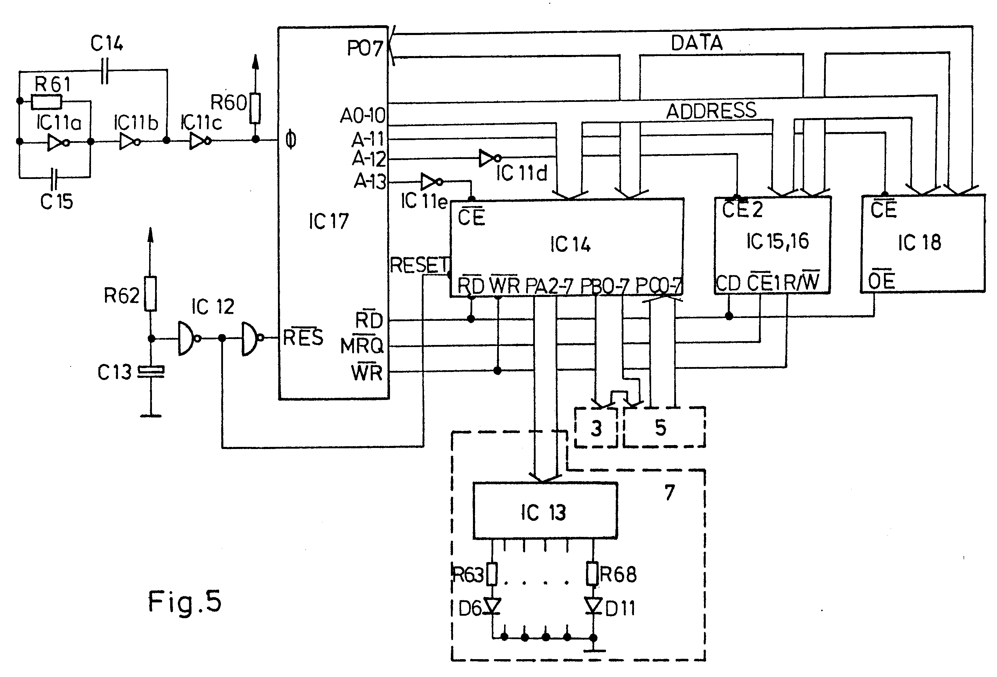

In Fig. 5 sind die mit einem Mikroprozessor ausgebildete Steuereinheit 6 und die Anzeigeeinheit 7 näher dargestellt. An den Taktsignaleingang eines Mikroprozessors IC17 ist der Ausgang eines Taktsignalgenerators, welcher mit Invertern IC11a-IC11c, Widerständen R60 und R61 und Kondensatoren C14 und C15 ausgebildet ist, angeschlossen, während an den Reseteingang R des Mikroprozessors IC17 ein Ausgang eines mit Invertern IC12, einem Widerstand R62 und einem Kondensator C13 ausgebildeten Autoreset-Stromkreises geführt ist.

An den Daten- und Adressenbus des Mikroprozessors IC17 sind eine Peripherie-Interfaceeinheit IC14, ein RAM-Speicher IC15,16 und ein ROM-Speicher IC18 angeschlossen. Die die Auswahl und Funktion der einzelnen Elemente steuernden Signale sind entsprechend den in den Katalogen empfohlenen Schaltungen an die entsprechenden Steuereingänge geführt. Bei der vorliegenden Ausführungsform werden beispielsweise ein Mikroprozessor des Types Z80, eine programmierbare Peripherie-Interfaceeinheit /I/O PORT/ des Types 8255, ein RAM-Speicher des Types 2111 und ein ROM-Speicher des Types 2716 verwendet. Die erfindungsgemässe Vorrichtung kann jedoch auch mit anderen Einheiten, z.B. irgendeinem, einen RAM- und ROM-Speicher, sowie eine I/O PORT-Einheit aufweisenden Mikroprozessor ausgebildet werden. Die an die Peripherie-Interfaceeinheit IC14 angeschlossene Anzeigeeinheit 7 ist mit einer Antriebsschaltung IC13, sowie an deren Ausgänge über strombegrenzende Widerstände R63-R68 angeschlossenen LED-Dioden D6-D11 ausgebildet.

Bei Einschaltung der Speisespannung sorgt der mit Invertern IC12 ausgebildete Autoreset-Stromkreis dafür, dass der Mikroprozessor IC17 und die Peripherie-Interfaceeinheit IC14 in Ausgangsposition gestellt werden. Der Taktsignalgenerator liefert an den Taktsignaleingang des Mikroprozessors IC17 ein Taktsignal mit einer Frequenz von 1MHz. Das die Messung steuernde Programm des Mikroprozessors wird von dem ROM-Speicher IC18 gespeichert, während der RAM-Speicher IC15, IC16 die Messdaten abspeichern. Der Mikroprozessor steuert den mit Analogschaltern ausgebildeten Messpunktschalter 3, den Transistor T1, welcher das Steuersignal für das Haltesignal (Hold-Signal) des A/D-Wandlers 5 erzeugt, und die Anzeigeeinheit 7 über die Peripherie-Interfaceeinheit IC14.

Bei der vorliegenden Ausführungsform enthält die Anzeigeeinheit 7 sechs LED-Dioden, von denen fünf in den vier Ecken eines Quadrates und in dessen Mittelpunkt angeordnet sind. Von den in den vier Ecken des Quadrates angeordneten LED-Dioden leuchtet diejenige während der Prüfung rot auf, die der Meßsonde zugeordnet ist, in welcher eine Milchprobe aufzufinden ist, für welche Mastitis nachgewiesen werden kann. Wenn für keine der Milchproben Mastitis nachgewiesen wird, leuchtet die in der Mitte angeordnete LED-Diode mit andauernder grüner Ausstrahlung. Die sechste LED-Diode leuchtet bei Niederdrücken einer zur Kontrolle der Speisespannung der Vorrichtung dienenden Tase, wenn die Spannung der die Speisespannung erzeugenden Akkumulatoren zur Durchführung weiterer Messungen nicht mehr ausreichend ist.

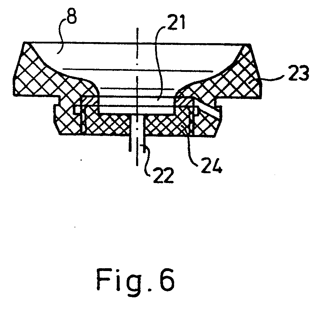

In Fig. 6 ist ein Ausführungsbeispiel einer in der erfindungsgemässen Vorrichtung verwendeten Meßsonde näher veranschaulicht, welche einen aus zwei, aneinander gewindeartig angepassten Kunststoffteilen bestehenden Messkelch 8 und im Messkelch befestigte Elektroden aufweist. Der Messkelch 8 ist mit einer aus Kunststoff bestehenden Kelchseite 23 und einem, ebenfalls aus Kunststoff bestehenden Kelchboden 24 ausgebildet. Zwischen der Kelchseite 23 und dem Kelchboden 24 ist eine Ringelektrode 21 eingefasst. In dem Kelchboden 24 ist eine axial angeordnete Stiftelektrode 22 befestigt, dessen Achsenende in der Ebene des Kelchbodens 24 liegt. Sowohl die Ringelektrode 21 als auch die Stiftelektrode 22 sind aus rostfreiem Stahl gefertigt.

Bei der Anwendung des erfindungsgemässen Verfahrens und der zur Durchführung des Verfahrens ausgebildeten Vorrichtung ist in grossen Milchwirtschaften bereits im subklinischen Stadium die Euterentzündung nachweisbar, wodurch die von gesunden und die von an Euterentzündung erkrankten Eutern stammende Milch voneinander getrennt werden kann, dadurch kann eine Verbesserung der Qualität der den Milchverarbeitungsbetrieben weitergeleiteten Milch erzielt werden, darüberhinaus wird die Behandlung der an Euterentzündung leidenden Kühe einfacher und erfolgreicher, da die Behandlung nach der Durchführung der Überprüfung schon in einem früheren Stadium begonnen werden kann.

Zur Durchführung der Kontrolle mittels der erfindungsgemässen Vorrichtung ist keine besondere Einführung oder Fachkenntnisse erforderlich und die Kontrolle kann innerhalb von Sekunden durchgeführt werden. Die Messzeit ist im Verhältnis zur Melkzeit vernachlässigbar gering, wodurch sogar eine 100 %-ige Messung pro Melke keinen Zeitverlust hervorruft, somit kann die Kontrolle in die Melktechnologie von Grossbetrieben eingepasst werden. Ein weiterer Vorteil des erfindungsgemässen Verfahrens gegenüber den bekannten Verfahren besteht darin, dass die Zuverlässigkeit des Nachweises grösser ist, welche bei den durchgeführten Probemessungen in allen Fällen über 80 % lag.

Patentansprüche

1. Verfahren zum Nachweis von Euterentzündung, bei welchem die Impedanz der aus den vier Eutervierteln des geprüften Subjektes abgemolkenen Milch gemessen wird und die auf diese Weise erhaltenen Werte mit einem im voraus bestimmten Wert verglichen werden, oder durch Teilung der erhaltenen Impedanzwerte durch den Impedanzwert der Milchprobe mit maximaler Impedanz je ein Quotient gebildet wird und die erhaltenen Quotienten mit einem im voraus bestimmten Wert verglichen werden, dadurch g e k e n n z e i c h n e t , daß die Messung der Impedanz in zwei Schritten durchgeführt wird, und zwar auf die Weise, dass der über die Milchprobe fliessende Strom und die über der Milchprobe abfallende Spannung gemessen werden, danach mittels einer zur Durchführung von arithmetischen Operationen ebenfalls geeigneten Steuerelektronik der Quotient der gemessenen Werte gebildet wird und die auf diese Weise erhaltenen Impedanzwerte gespeichert werden, dass die gleichen Operationen für alle vier, den vier Eutervierteln entnommenen Milchproben durchgeführt werden und der maximale Impedanzwert gesucht wird, danach die Quotienten der Impedanzen der übrigen Milchproben zur maximalon Impedanz gebildet werden und die auf diese Weise erhaltenen Quotienten mit einem solchen Grenzwert verglichen werden, dessen Wert in Abhängigkeit vom Impedanzwert der Milchprobe mit maximaler Impedanz im voraus auf die Weise bestimmt wurde, dass die möglichen Impedanzwert in mehrere Bereiche aufgeteilt werden und jedem Bereich ein experimentell ermittelter Grenzimpedanzquotient zugeordnet wird, wobei die Milchprobe, deren Impedanzquotient kleiner als der den einzelnen Bereichen zugeordnete Grenzimpedanzquotient ist, als mit Euterentzündung behaftete bewertet wird.

2. Vorrichtung zum Nachweis von Euterentzündung, welche mit mindestens einer Meßsonde, einer elektronischen Einheit und einer Anzeigeeinheit versehen ist, dadurch gekennzeichnet, dass die elektronische Einheit mit einem, ein Wechselspannungssignal erzeugenden Generator (1), einem Meßverstärker (4), einem zwischen den Meßsonden und dem Messverstärker eingefügten Messpunktschalter - vorzugsweise einem Analogschalter -, einem das analoge Signal des Messverstärkers in ein digitales Signal unwandelnden A/D-Wandler (5) und einer mit einem Mikroprozessor versehenen Steuereinheit (6) ausgebildet ist, desweiteren die Meßsonden (2) mit dem Ausgang des Generators (1), ein ein analoges Signal liefernder Ausgang des Messverstärkers (4) mit dem Eingang des A/D-Wandlers (5), die digitalen Ausgänge des A/D-Wandlers (5) mit Eingängen der Steuereinheit (6), Steuerausgänge der Steuereinheit (6) mit den Steuereingängen der Messpunktschalter (3) und den Eingängen der Anzeigeeinheit (7) verbunden sind.

3. Vorrichtung nach Anspruch 2, dadurch gekennzeichnet, daß die Meßsonde (2) mit einem aus Isolationsmaterial - vorzugsweise Kunststoff - bestehenden Messkelch (8) und in dem Messkelch befestigten Elektroden (21,22) ausgebildet ist, wobei der Messkelch von einer Kelchseite (23) und einem Kelchboden (24) begrenzt ist, desweiteren zwischen der Kelchseite (23) und dem Kelchboden (24) eine Ringelektrode (21) angeordnet ist, während im Kelchboden (24) eine zur Ebene der Ringelektrode im wesentlichen quergerichtete, in deren Achse angeordnete Stiftelektrode (22) befestigt ist.

4. Vorrichtung nach Anspruch 3, dadurch gekennzeichnet, daß der aus Kunststoff bestehende Messkelch (8) aus zwei einander gewindeartig angepaßten Teilen besteht.

Fig.1

0211386

Fig.2

Fig.3

Fig.4

Fig.5

-5-

0211386

Fig. 6